(19) Europäisches Patentamt / European Patent Office / Office européen des brevets

(11) **EP 2 610 639 A2**

(12) **EUROPEAN PATENT APPLICATION**

(43) Date of publication:
**03.07.2013 Bulletin 2013/27**

(51) Int Cl.:
**G01S 15/89** $^{(2006.01)}$     **G01S 7/52** $^{(2006.01)}$

(21) Application number: **12187919.1**

(22) Date of filing: **10.10.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **28.12.2011 KR 20110144489**

(71) Applicant: **Samsung Medison Co., Ltd.**
**Gangwon-do 250-870 (KR)**

(72) Inventors:
• **Kim, Min Woo**
  **135-851 Seoul (KR)**
• **Kim, Hyoung Jin**
  **135-851 Seoul (KR)**

(74) Representative: **Schmid, Wolfgang**
**Lorenz & Kollegen**
**Patentanwälte Partnerschaftsgesellschaft**
**Alte Ulmer Strasse 2**
**89522 Heidenheim (DE)**

(54) **Estimating motion of particle based on vector doppler in ultrasound system**

(57)    There are provided embodiments for estimating the motion of at least one particle by using vector Doppler. In one embodiment, by way of non-limiting example, an ultrasound system comprises: a processing unit configured to form vector information of a target object based on ultrasound data corresponding to the target object, form a plurality of Doppler mode images based on the vector information, set at least one particle on the Doppler mode images based on input information of a user, and estimate a motion of the at least one particle based on the vector information.

## FIG. 1

**Description**

CROSS-REFERENCE TO RELATED APPLICATIONS

The present application claims priority from Korean Patent Application No. 10-2011-0144489 filed on December 28, 2011.

TECHNICAL FIELD

The present disclosure generally relates to ultrasound systems, and more particularly to estimating the motion of at least one particle by using vector Doppler in an ultrasound system.

BACKGROUND

An ultrasound system has become an important and popular diagnostic tool since it has a wide range of applications. Specifically, due to its non-invasive and non-destructive nature, the ultrasound system has been extensively used in the medical profession. Modern high-performance ultrasound systems and techniques are commonly used to produce two-dimensional or three-dimensional ultrasound images of internal features of target objects (e.g., human organs).

The ultrasound system provides ultrasound images of various modes including a brightness mode image representing reflection coefficients of ultrasound signals (i.e., ultrasound echo signals) reflected from a target object of a living body with a two-dimensional image, a Doppler mode image representing velocity of a moving target object with spectral Doppler by using a Doppler effect, a color Doppler mode image representing velocity of the moving target object with colors by using the Doppler effect, an elastic image representing mechanical characteristics of tissues before and after applying compression thereto, etc.

The ultrasound system transmits ultrasound signals to the living body including a moving target object (e.g., blood flow) and receives ultrasound signals (i.e., ultrasound echo signals) from the living body. The ultrasound system further forms the color Doppler mode image representing velocities of the target object with colors based on the ultrasound echo signals. The color Doppler image is used to diagnose disease of a blood vessel, a heart and the like. However, the color Doppler image cannot represent an accurate motion of the target object since the respective colors in the color Doppler image indicate the velocity of the target object, which moves forward in a transmission direction of the ultrasound signals and backward in the transmission direction of the ultrasound signals.

To resolve this problem, vector Doppler methods capable of obtaining motion (i.e., velocity and direction) of the target object are used. A cross beam-based method of the vector Doppler methods acquires velocity components of the target object from at least two different directions, and combines the velocity components to form vector information including two-dimensional or three-dimensional direction information and velocity information.

SUMMARY

There are provided embodiments for estimating the motion of at least one particle by using vector Doppler.

In one embodiment, by way of non-limiting example, an ultrasound system comprises: a processing unit configured to form vector information of a target object based on ultrasound data corresponding to the target object, form a plurality of Doppler mode images based on the vector information, set at least one particle on the Doppler mode images based on input information of a user, and estimate a motion of the at least one particle based on the vector information.

In another embodiment, there is provided a method of estimating the motion of at least one particle, comprising: a) forming vector information of a target object based on ultrasound data corresponding to the target object; b) forming a plurality of Doppler mode images based on the vector information; c) setting at least one particle on the Doppler mode images based on input information of a user; and d) estimating a motion of the at least one particle based on the vector information.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

BRIEF DESCRIPTION OF THE DRAWINGS

FIG. 1 is a block diagram showing an illustrative embodiment of an ultrasound system.

FIG. 2 is a schematic diagram showing an example of a brightness mode image and a region of interest.

FIG. 3 is a block diagram showing an illustrative embodiment of an ultrasound data acquiring unit.

FIGS. 4 to 7 are schematic diagrams showing examples of transmission directions and reception directions.

FIG. 8 is a schematic diagram showing an example of sampling data and pixels of an ultrasound image.

FIGS. 9 to 12 are schematic diagrams showing examples of performing a receiving beam-forming.

FIG. 13 is a schematic diagram showing an example of setting weights.

FIG. 14 is a schematic diagram showing an example of setting a sampling data set.

FIG. 15 is a flow chart showing a process of estimating motion of at least one particle.

FIG. 16 is a schematic diagram showing an example of the transmission directions, the reception directions, the vector information and an over-determined problem.

FIG. 17 is a schematic diagram showing an example of setting the at least one particle.

FIG. 18 is a schematic diagram showing an example of estimating a streamline.

FIG. 19 is a schematic diagram showing an example of moving the particle.

## DETAILED DESCRIPTION

A detailed description is provided with reference to the accompanying drawings. One of ordinary skill in the art should realize that the following description is illustrative only and is not in any way limiting.

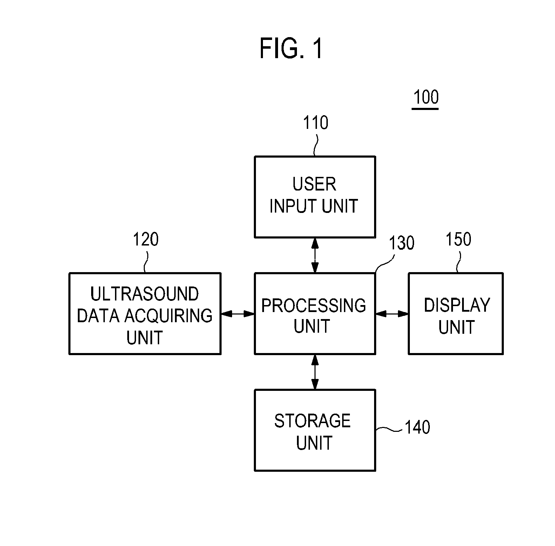

Referring to FIG. 1, an ultrasound system 100 in accordance with an illustrative embodiment is shown. As depicted herein, the ultrasound system 100 includes a user input unit 110.

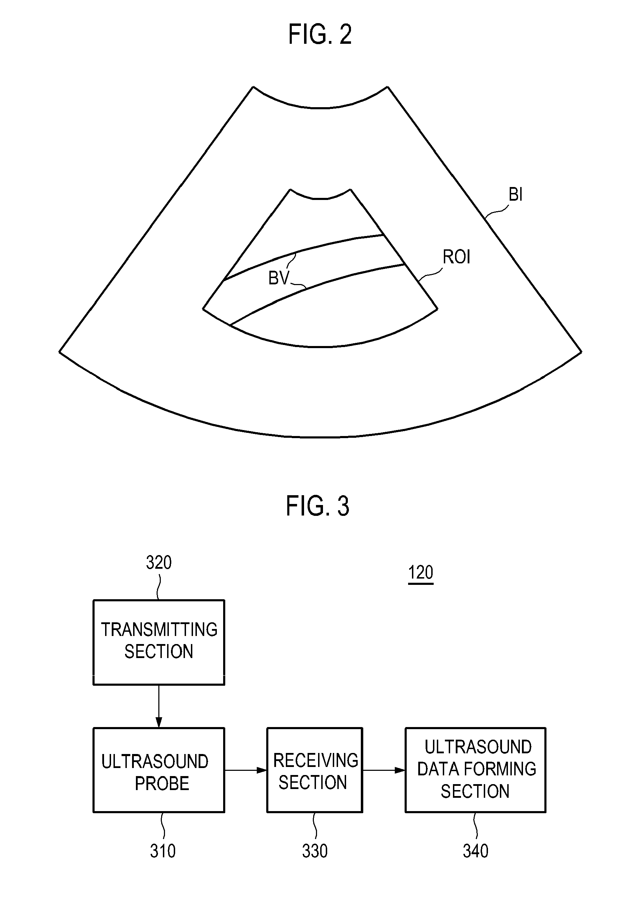

The user input unit 110 is configured to receive input information from a user. In one embodiment, the input information includes first input information for setting a region of interest ROI on a brightness mode image BI, as shown in FIG. 2. The region of interest ROI includes a color box for obtaining a Doppler mode image. The Doppler mode image includes a vector Doppler image or a color Doppler image. However, it should be noted herein that the Doppler mode image may not be limited thereto. In FIG. 2, a reference numeral BV represents a blood vessel. The input information further includes second input information for setting at least one particle on the Doppler mode image. That is, the second input information includes information for setting the number, position, size, color and shape of the particle, an alignment position between the particles and the like. The user input unit 110 includes a control panel, a track ball, a mouse, a keyboard and the like.

The ultrasound system 100 further includes an ultrasound data acquiring unit 120. The ultrasound data acquiring unit 120 is configured to transmit ultrasound signals to a living body. The living body includes moving target objects (e.g., blood vessel, heart, blood flow, etc). The ultrasound data acquiring unit 120 is further configured to receive ultrasound signals (i.e., ultrasound echo signals) from the living body to acquire ultrasound data corresponding to an ultrasound image.

FIG. 3 is a block diagram showing an illustrative embodiment of the ultrasound data acquiring unit. Referring to FIG. 3, the ultrasound data acquiring unit 120 includes an ultrasound probe 310.

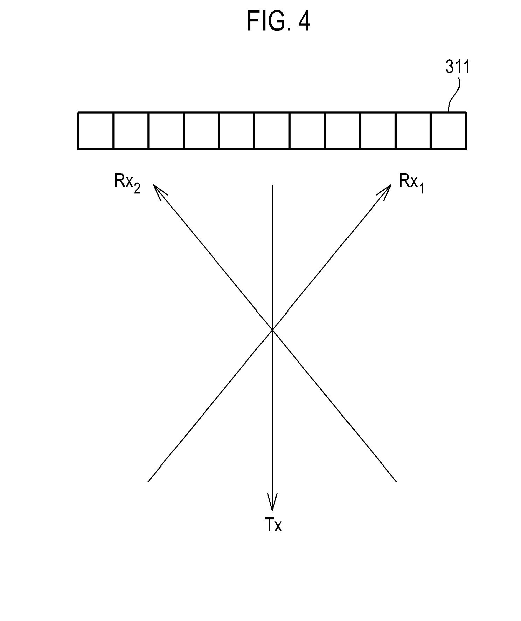

The ultrasound probe 310 includes a plurality of elements 311 (*see* FIG. 4) for reciprocally converting between ultrasound signals and electrical signals. The ultrasound probe 310 is configured to transmit the ultrasound signals to the living body. The ultrasound signals transmitted from the ultrasound probe 310 are plane wave signals that the ultrasound signals are not focused at a focusing point or focused signals that the ultrasound signals are focused at the focusing point. However, it should be noted herein that the ultrasound signals may not be limited thereto. The ultrasound probe 310 is further configured to receive the ultrasound echo signals from the living body to output electrical signals (hereinafter, referred to as "reception signals"). The reception signals are analog signals. The ultrasound probe 310 includes a convex probe, a linear probe and the like.

The ultrasound data acquiring unit 120 further includes a transmitting section 320. The transmitting section 320 is configured to control the transmission of the ultrasound signals. The transmitting section 320 is further configured to generate electrical signals (hereinafter, referred to as "transmission signals") in consideration of the elements 311.

In one embodiment, the transmitting section 320 is configured to generate transmission signals (hereinafter, referred to as "brightness mode transmission signals") for obtaining the brightness mode image BI in consideration of the elements 311. Thus, the ultrasound probe 310 is configured to convert the brightness mode transmission signals provided from the transmitting section 320 into the ultrasound signals, transmit the ultrasound signals to the living body, and receive the ultrasound echo signals from the living body to output reception signals (hereinafter, referred to as "brightness mode reception signals").

The transmitting section 320 is further configured to generate transmission signals (hereinafter, referred to as "Doppler mode transmission signals") corresponding to an ensemble number in consideration of the elements 311 and at least one transmission direction of the ultrasound signals (i.e., transmission beam). Thus, the ultrasound probe 310 is configured to convert the Doppler mode transmission signals provided from the transmitting section 320 into the ultrasound signals, transmit the ultrasound signals to the living body in the at least one transmission direction, and receive the ultrasound echo signals from the living body to output reception signals (hereinafter, referred to as "Doppler mode reception signals"). The ensemble number represents the number of transmitting and receiving the ultrasound signals to/from a target object.

As one example, the transmitting section 320 is configured to generate the Doppler mode transmission signals corresponding to the ensemble number in consideration of a transmission direction Tx and the elements 311, as shown in FIG. 4. The transmission direction is one direction in the range of a direction (0 degree) perpendicular to a longitudinal direction of the elements 311 to a maximum steering direction of the transmission beam.

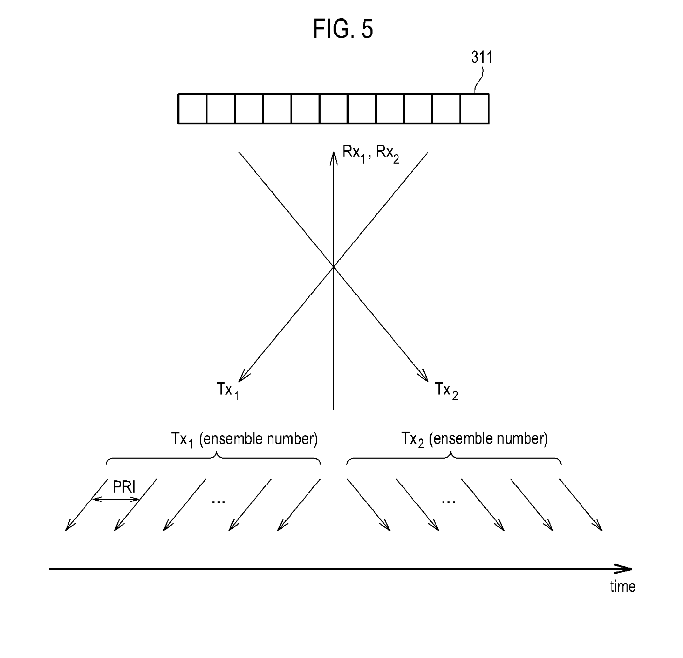

As another example, the transmitting section 320 is configured to generate first Doppler mode transmission signals corresponding to the ensemble number in consideration of a first transmission direction $Tx_1$ and the elements 311, as shown in FIG. 5. Thus, the ultrasound probe 310 is configured to convert the first Doppler mode transmission signals provided from the transmitting section 320 into the ultrasound signals, transmit the ultrasound signals to the living body in the first transmission direction $Tx_1$, and receive the ultrasound echo signals from the living body to output first Doppler mode reception signals. The transmitting section 320 is further configured to generate second Doppler mode transmission signals corresponding to the ensemble number in consideration of a second transmission direction $Tx_2$ and the elements 311, as shown in FIG. 5. Thus, the ultrasound probe 310 is configured to convert the second Doppler mode transmission signals provided from the transmitting section 320 into the ultrasound signals, transmit the ultrasound signals to the living body in the second transmission direction $Tx_2$, and receive the ultrasound echo signals from the living body to output second Doppler mode reception signals. In FIG. 5, a reference numeral PRI represents a pulse repeat interval.

In another embodiment, the transmitting section 320 is configured to generate the brightness mode transmission signals for obtaining the brightness mode image BI in consideration of the elements 311. Thus, the ultrasound probe 310 is configured to convert the brightness mode transmission signals provided from the transmitting section 320 into the ultrasound signals, transmit the ultrasound signals to the living body, and receive the ultrasound echo signals from the living body to output the brightness mode reception signals.

The transmitting section 320 is further configured to generate the Doppler mode transmission signals corresponding to the ensemble number in consideration of the at least one transmission direction and the elements 311. Thus, the ultrasound probe 310 is configured to convert the Doppler mode transmission signals provided from the transmitting section 320 into the ultrasound signals, transmit the ultrasound signals to the living body, and receive the ultrasound echo signals from the living body to output the Doppler mode reception signals. The ultrasound signals are transmitted in an interleaved transmission scheme. The interleaved transmission scheme will be described below in detail.

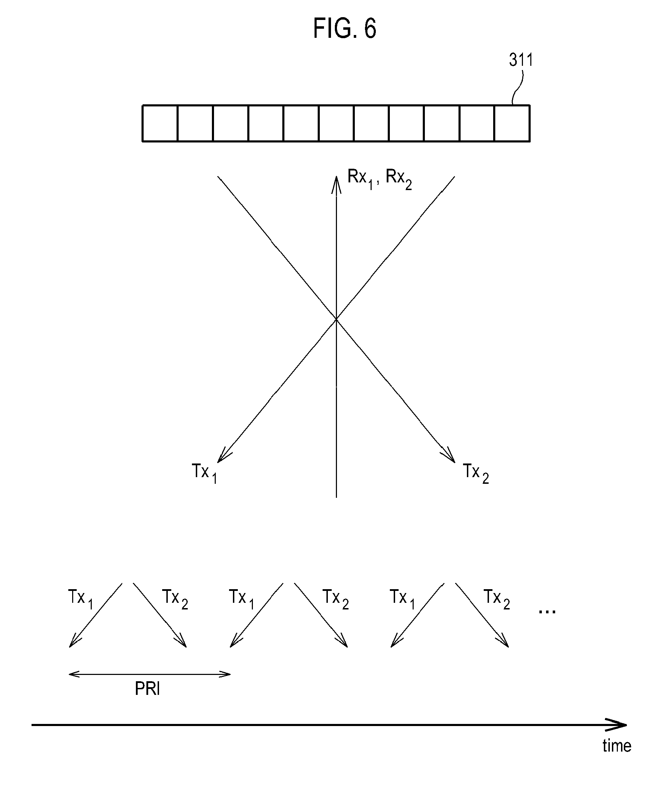

For example, the transmitting section 320 is configured to generate the first Doppler mode transmission signals in consideration of the first transmission direction $Tx_1$ and the elements 311, as shown in FIG. 6. Thus, the ultrasound probe 310 is configured to convert the first Doppler mode transmission signals provided from the transmitting section 320 into the ultrasound signals, and transmit the ultrasound signals to the living body in the first transmission direction $Tx_1$. Thereafter, the transmitting section 320 is further configured to generate the second Doppler mode transmission signals in consideration of the second transmission direction $Tx_2$ and the elements 311, as shown in FIG. 6. Thus, the ultrasound probe 310 is configured to convert the second Doppler mode transmission signals provided from the transmitting section 320 into the ultrasound signals, and transmit the ultrasound signals to the living body in the second transmission direction $Tx_2$. The ultrasound probe 310 is further configured to receive the ultrasound echo signals (i.e., ultrasound echo signals corresponding to first Doppler mode transmission signals) from the living body to output the first Doppler mode reception signals. The ultrasound probe 310 is further configured to receive the ultrasound echo signals (i.e., ultrasound echo signals corresponding to second Doppler mode transmission signals) from the living body to output the second Doppler mode reception signals.

Thereafter, the transmitting section 320 is configured to generate the first Doppler mode transmission signals based on the pulse repeat interval, as shown in FIG. 6. Thus, the ultrasound probe 310 is configured to convert the first Doppler mode transmission signals provided from the transmitting section 320 into the ultrasound signals, and transmit the ultrasound signals to the living body in the first transmission direction $Tx_1$. The transmitting section 320 is further configured to generate the second Doppler mode transmission signals based on the pulse repeat interval, as shown in FIG. 6. Thus, the ultrasound probe 310 is configured to convert the second Doppler mode transmission signals provided from the transmitting section 320 into the ultrasound signals, and transmit the ultrasound signals to the living body in the second transmission direction $Tx_2$. The ultrasound probe 310 is further configured to receive the ultrasound echo signals (i.e., ultrasound echo signals corresponding to first Doppler mode transmission signals) from the living body to output the first Doppler mode reception signals. The ultrasound probe 310 is further configured to receive the ultrasound echo signals (i.e., ultrasound echo signals corresponding to second Doppler mode transmission signals) from the living body to output the second Doppler mode reception signals.

As described above, the transmitting section 320 is configured to generate the first Doppler mode transmission signals and the second Doppler mode transmission signals corresponding to the ensemble number.

In yet another embodiment, the transmitting section 320 is configured to generate the brightness mode transmission signals for obtaining the brightness mode image BI in consideration of the elements 311. Thus, the ultrasound probe 310 is configured to convert the brightness mode transmission signals provided from the transmitting section 320 into the ultrasound signals, transmit the ultrasound signals to the living body, and receive the ultrasound echo signals from the living body to output the brightness mode reception signals.

The transmitting section 320 is further configured to generate the Doppler mode transmission signals corresponding to the ensemble number in consideration of the at least one transmission direction and the elements 311. Thus, the ultrasound probe 310 is configured to convert the Doppler mode transmission signals provided from the transmitting section 320 into the ultrasound signals, transmit the ultrasound signals to the living body, and receive the ultrasound echo signals from the living body to output the Doppler mode reception signals. The ultrasound signals are transmitted according to the pulse repeat interval.

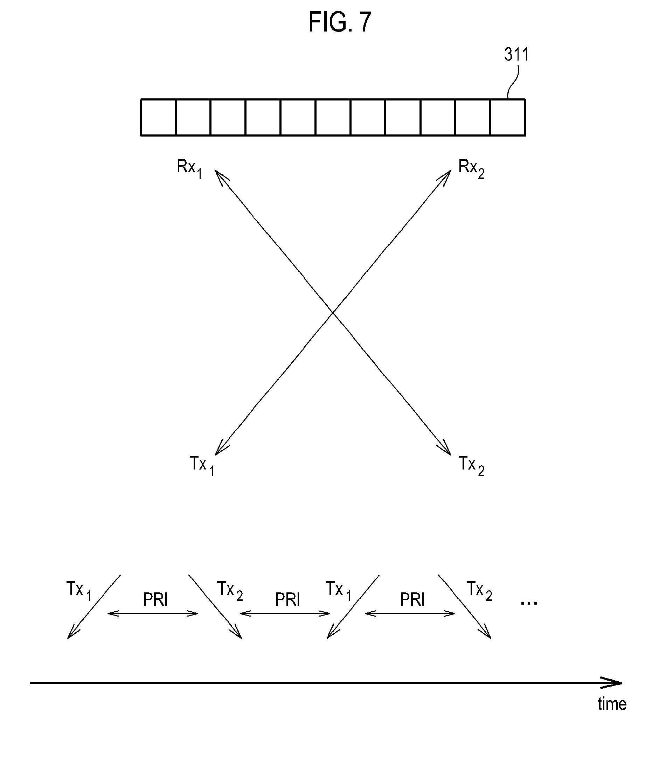

For example, the transmitting section 320 is configured to generate the first Doppler mode transmission signals in consideration of the first transmission direction $Tx_1$ and the elements 311 based on the pulse repeat interval, as shown in FIG. 7. As such, the ultrasound probe 310 is configured to convert the first Doppler mode transmission signals provided from the transmitting section 320 into the ultrasound signals, transmit the ultrasound signals to living body in the first transmission direction $Tx_1$, and receive the ultrasound echo signals from the living body to output the first Doppler mode reception signals. The transmitting section 320 is further configured to generate the second Doppler mode transmission signals in consideration of the second transmission direction $Tx_2$ and the elements 311, based on the pulse repeat interval, as shown in FIG. 7. Thus, the ultrasound probe 310 is configured to convert the second Doppler mode transmission signals provided from the transmitting section 320 into the ultrasound signals, transmit the ultrasound signals to the living body in the second transmission direction $Tx_2$, and receive the ultrasound echo signals from the living body to output the second Doppler mode reception signals.

As described above, the transmitting section 320 is configured to generate the first Doppler mode transmission signals and the second Doppler mode transmission signals corresponding to the ensemble number based on the pulse repeat interval.

Referring back to FIG. 3, the ultrasound data acquiring unit 120 further includes a receiving section 330. The receiving section 330 is configured to perform an analog-digital conversion upon the reception signals provided from the ultrasound probe 310 to form sampling data of the reception signals. The receiving section 330 is further configured to perform a reception beam-forming upon the sampling data in consideration of the elements 311 to form reception-focused data. The reception beam-forming will be described below in detail.

In one embodiment, the receiving section 330 is configured to perform the analog-digital conversion upon the brightness mode reception signals provided from the ultrasound probe 310 to form sampling data (hereinafter, referred to as "brightness mode sampling data"). The receiving section 330 is further configured to perform the reception beam-forming upon the brightness mode sampling data to form reception-focused data (hereinafter, referred to as "brightness mode reception-focused data").

The receiving section 330 is further configured to perform the analog-digital conversion upon the Doppler mode reception signals provided from the ultrasound probe 310 to form sampling data (hereinafter, referred to as "Doppler mode sampling data"). The receiving section 330 is further configured to perform the reception beam-forming upon the Doppler mode sampling data to form reception-focused data (hereinafter, referred to as "Doppler mode reception-focused data") corresponding to the at least one reception direction of the ultrasound echo signals (i.e., reception beam).

As one example, the receiving section 330 is configured to perform the analog-digital conversion upon the Doppler mode reception signals provided from the ultrasound probe 310 to form the Doppler mode sampling data. The receiving section 330 is further configured to perform the reception beam-forming upon the Doppler mode sampling data to form first Doppler mode reception-focused data corresponding to the first reception direction $Rx_1$ and second Doppler mode reception-focused data corresponding to the second reception direction $Rx_2$, as shown in FIG. 4.

As another example, the receiving section 330 is configured to perform the analog-digital conversion upon the first Doppler mode reception signals provided from the ultrasound probe 310 to form first Doppler mode sampling data corresponding to the first transmission direction $Tx_1$, as shown in FIG. 5. The receiving section 330 is further configured to perform the reception beam-forming upon the first Doppler mode sampling data to form the first Doppler mode reception-focused data corresponding to the first reception direction $Rx_1$. The receiving section 330 is further configured to perform the analog-digital conversion upon the second Doppler mode reception signals provided from the ultrasound probe 310 to form second Doppler mode sampling data corresponding to the second transmission direction $Tx_2$, as shown in FIG. 5. The receiving section 330 is further configured to perform the reception beam-forming upon the second Doppler mode sampling data to form the second Doppler mode reception-focused data corresponding to the second reception direction $Rx_2$. If the reception direction is perpendicular to the elements 311 of the ultrasound probe 310, then an aperture size of being capable of receiving the ultrasound signals can be a maximum value.

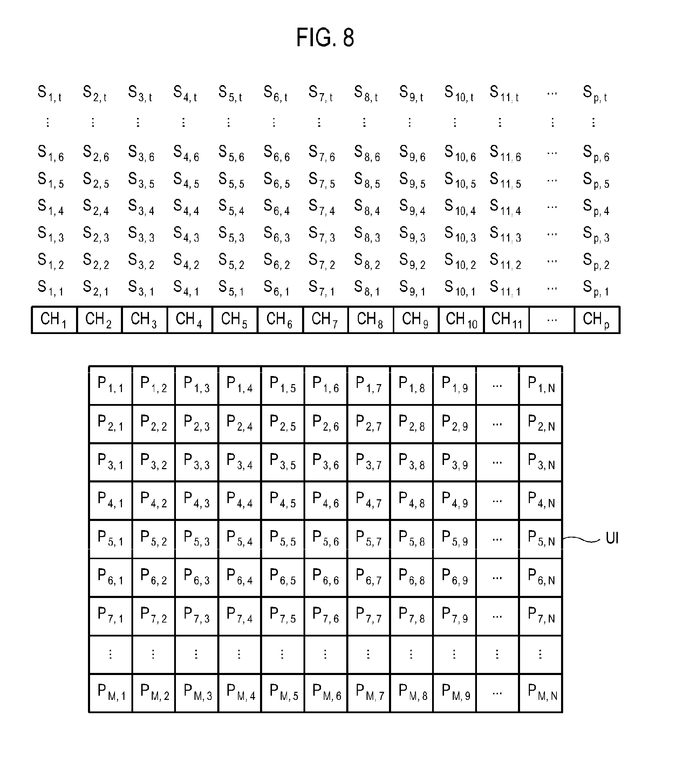

The reception beam-forming is described with reference to the accompanying drawings. In one embodiment, the receiving section 330 is configured to perform the analog-digital conversion upon the reception signals provided through a plurality of channels $CH_k$, wherein $1 \leq k \leq N$, from the ultrasound probe 310 to form sampling data $S_{i,j}$, wherein the i and j are a positive integer, as shown in FIG. 8. The sampling data $S_{i,j}$ are stored in a storage unit 140. The receiving section 330 is further configured to detect pixels corresponding to the sampling data based on positions of the elements 311 and orientation of pixels $P_{a,b}$, wherein $1 \le a \le M$, $1 \le b \le N$, of the ultrasound image UI with respect to the elements 311. That is, the receiving section 330 selects the pixels that the respective sampling data are used as pixel data thereof, during the reception beam-forming based on the positions of the elements 311 and the orientation of the respective pixels of the ultrasound image UI with respect to the elements 311. The receiving section 330 is further configured to cumulatively assign the sampling data corresponding to the selected pixels as the pixel data.

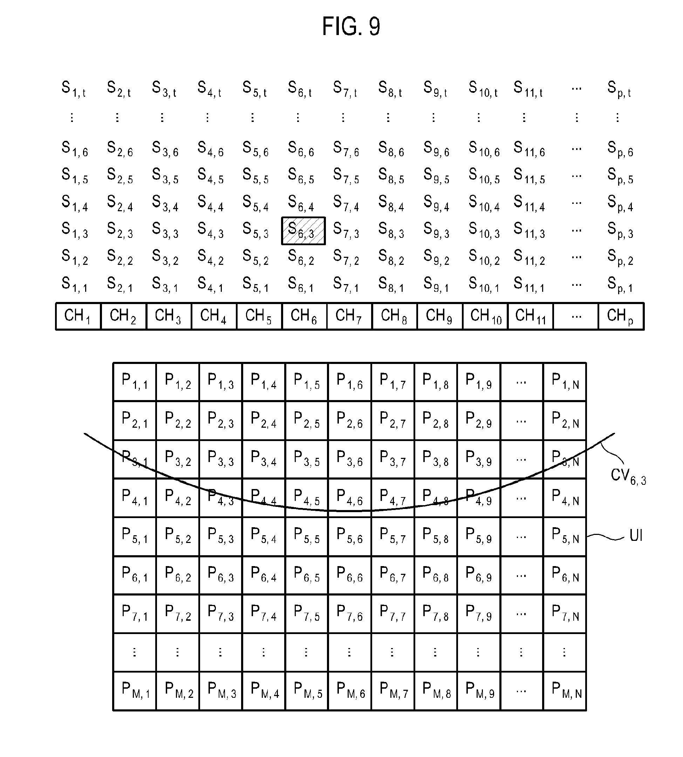

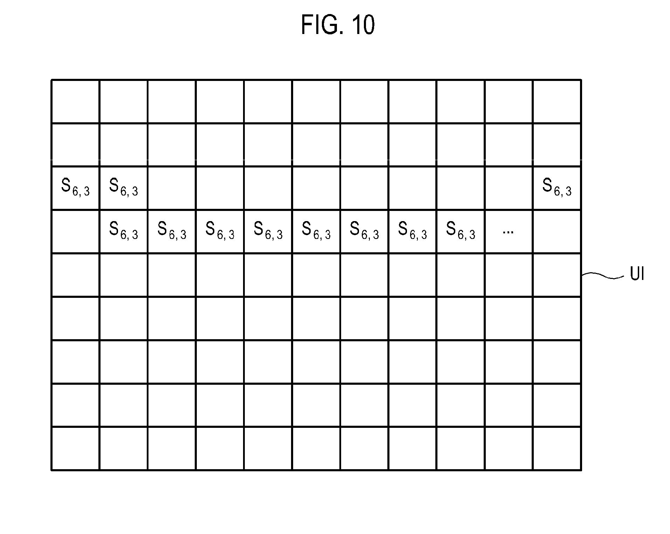

For example, the receiving section 330 is configured to set a curve (hereinafter, referred to as "reception beam-forming curve") $CV_{6,3}$ for selecting pixels that the sampling data $S_{6,3}$ are used as the pixel data thereof, during the reception beam-forming based on the positions of the elements 311 and the orientation of the respective pixels of the ultrasound image UI with respect to the elements 311, as shown in FIG. 9. The receiving section 330 is further configured to detect the pixels $P_{3,1}$, $P_{3,2}$, $P_{4,2}$, $P_{4,3}$, $P_{4,4}$, $P_{4,5}$, $P_{4,6}$, $P_{4,7}$, $P_{4,8}$, $P_{4,9}$, ... $P_{3,N}$ corresponding to the reception beam-forming curve $CV_{6,3}$ from the pixels $P_{a,b}$ of the ultrasound image UI, wherein $1 \le a \le M$, $1 \le b \le N$. That is, the receiving section 330 selects the pixels $P_{3,1}$, $P_{3,2}$, $P_{4,2}$, $P_{4,3}$, $P_{4,4}$, $P_{4,5}$, $P_{4,6}$, $P_{4,7}$, $P_{4,8}$, $P_{4,9}$, ... $P_{3,N}$ on which the reception beam-forming curve $CV_{6,3}$ passes among the pixels $P_{a,b}$ of the ultrasound image UI. The receiving section 330 is further configured to assign the sampling data $S_{6,3}$ to the selected pixels $P_{3,1}$, $P_{3,2}$, $P_{4,2}$, $P_{4,3}$, $P_{4,4}$, $P_{4,5}$, $P_{4,6}$, $P_{4,7}$, $P_{4,8}$, $P_{4,9}$, ... $P_{3,N}$, as shown in FIG. 10.

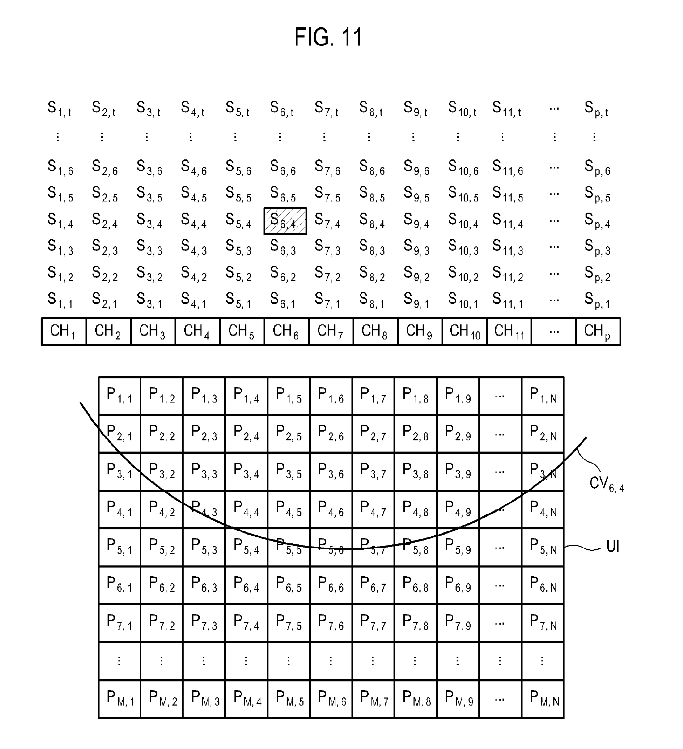

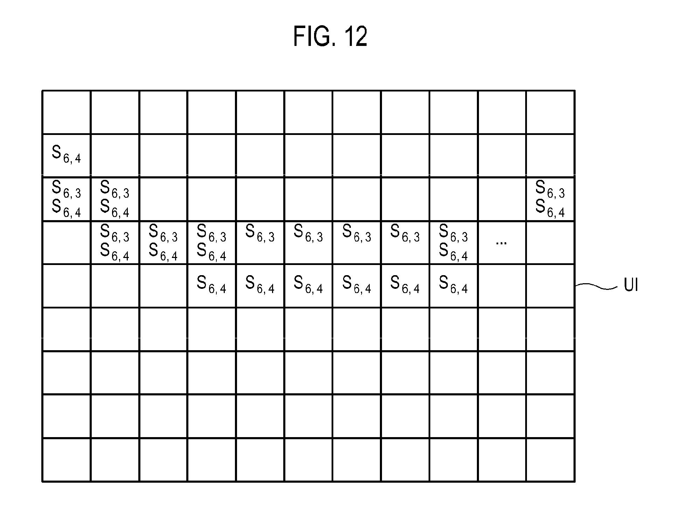

Thereafter, the receiving section 330 is configured to set a reception beam-forming curve $CV_{6,4}$ for selecting pixels that the sampling data $S_{6,4}$ are used as the pixel data thereof, during the reception beam-forming based on the positions of the elements 311 and the orientation of the respective pixels of the ultrasound image UI with respect to the elements 311, as shown in FIG. 11. The receiving section 330 is further configured to detect the pixels $P_{2,1}$, $P_{3,1}$, $P_{3,2}$, $P_{4,2}$, $P_{4,3}$, $P_{4,4}$, $P_{5,4}$, $P_{5,5}$, $P_{5,6}$, $P_{5,7}$, $P_{5,8}$, $P_{4,9}$, $P_{5,9}$, ... $P_{4,N}$, $P_{3,N}$ corresponding to the reception beam-forming curve $CV_{6,4}$ from the pixels $P_{a,b}$ of the ultrasound image UI. That is, the receiving section 330 selects the pixels $P_{2,1}$, $P_{3,1}$, $P_{3,2}$, $P_{4,2}$, $P_{4,3}$, $P_{4,4}$, $P_{5,4}$, $P_{5,5}$, $P_{5,6}$, $P_{5,7}$, $P_{5,8}$, $P_{4,9}$, $P_{5,9}$, ... $P_{4,N}$, $P_{3,N}$ on which the reception beam-forming curve $CV_{6,4}$ passes among the pixels $P_{a,b}$ of the ultrasound image UI. The receiving section 330 is further configured to assign the sampling data $S_{6,4}$ to the Selected pixels $P_{2,1}$, $P_{3,1}$, $P_{3,2}$, $P_{4,2}$, $P_{4,3}$, $P_{4,4}$, $P_{5,4}$, $P_{5,5}$, $P_{5,6}$, $P_{5,7}$, $P_{5,8}$, $P_{4,9}$, $P_{5,9}$, ... $P_{4,N}$, $P_{3,N}$, as shown in FIG. 12. In this way, the respective sampling data, which are used as the pixel data, are cumulatively assigned to the pixels as the pixel data.

The receiving section 330 is configured to perform the reception beam-forming (i.e., summing) upon the sampling data which are cumulatively assigned to the respective pixels $P_{a,b}$ of the ultrasound image UI to form the reception-focused data.

In another embodiment, the receiving section 330 is configured to perform the analog-digital conversion upon the reception signals provided through the plurality of channels $CH_k$ from the ultrasound probe 310 to form the sampling data $S_{i,j}$, as shown in FIG. 8. The sampling data $S_{i,j}$ are stored in the storage unit 140. The receiving section 330 is further configured to detect pixels corresponding to the sampling data based on the positions of the elements 311 and the orientation of the pixels of the ultrasound image UI with respect to the elements 311. That is, the receiving section 330 selects the pixels that the respective sampling data are used as the pixel data thereof, during the reception beam-forming based on the positions of the elements 311 and the orientation of the respective pixels of the ultrasound image UI with respect to the elements 311. The receiving section 330 is configured to cumulatively assign the sampling data corresponding to the selected pixels as the pixel data. The receiving section 330 is further configured to determine pixels existing in the same column among the selected pixels. The receiving section 330 is further configured to set weights corresponding to the respective determined pixels. The receiving section 330 is further configured to apply the weights to the sampling data of the respective pixels.

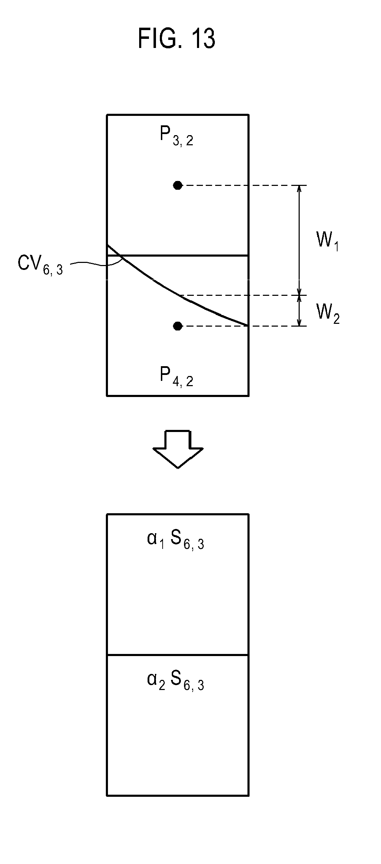

For example, the receiving section 330 is configured to set the reception beam-forming curve $CV_{6,3}$ for selecting pixels that the sampling data $S_{6,3}$ are used as the pixel data thereof, during the reception beam-forming based on the positions of the elements 311 and the orientation of the respective pixels of the ultrasound image UI with respect to the elements 311, as shown in FIG. 9. The receiving section 330 is further configured to detect the pixels $P_{3,1}$, $P_{3,2}$, $P_{4,2}$, $P_{4,3}$, $P_{4,4}$, $P_{4,5}$, $P_{4,6}$, $P_{4,7}$, $P_{4,8}$, $P_{4,9}$, ... $P_{3,N}$ corresponding to the reception beam-forming curve $CV_{6,3}$ from the pixels $P_{a,b}$ of the ultrasound image UI. That is, the receiving section 330 selects the pixels $P_{3,1}$, $P_{3,2}$, $P_{4,2}$, $P_{4,3}$, $P_{4,4}$, $P_{4,5}$, $P_{4,6}$, $P_{4,7}$, $P_{4,8}$, $P_{4,9}$, ... $P_{3,N}$ on which the reception beam-forming curve $CV_{6,3}$ passes among the pixels $P_{a,b}$ of the ultrasound image UI. The receiving section 330 is further configured to assign the sampling data $S_{6,3}$ to the selected pixels $P_{3,1}$, $P_{3,2}$, $P_{4,2}$, $P_{4,3}$, $P_{4,4}$, $P_{4,5}$, $P_{4,6}$, $P_{4,7}$, $P_{4,8}$, $P_{4,9}$, ... $P_{3,N}$, as shown in FIG. 10. The receiving section 330 is further configured to determine pixels $P_{3,2}$ and $P_{4,2}$ which exist in the same column among the selected pixels $P_{3,1}$, $P_{3,2}$, $P_{4,2}$, $P_{4,3}$, $P_{4,4}$, $P_{4,5}$, $P_{4,6}$, $P_{4,7}$, $P_{4,8}$, $P_{4,9}$, ... $P_{3,N}$. The receiving section 330 is further configured to calculate a distance $W_1$ from a center of the determined pixel $P_{3,2}$ to the reception beam-forming curve $CV_{6,3}$ and a distance $W_2$ from a center of the determined pixel $P_{4,2}$ to the reception beam-forming curve $CV_{6,3}$, as shown in FIG 13. The receiving section 330 is further configured to set a first weight $\alpha_1$ corresponding to the pixel $P_{3,2}$ based on the distance $W_1$ and a second weight $\alpha_2$ corresponding to the pixel $P_{4,2}$ based on the distance $W_2$. The first weight $\alpha_1$ and the second weight $\alpha_2$ are set to be in proportion to or in inverse proportion to the calculated distances. The receiving section 330 is further configured to apply the first weight $\alpha_1$ to the sampling data $S_{6,3}$ assigned to the pixel $P_{3,2}$ and to apply the second weight $\alpha_2$ to the sampling data $S_{6,3}$ assigned to the pixel $P_{4,2}$. The receiving section 330 is configured to perform the above process upon the remaining sampling data.

The receiving section 330 is configured to perform the reception beam-forming upon the sampling data which are cumulatively assigned to the respective pixels $P_{a,b}$ of the ultrasound image UI to form the reception-focused data.

In yet another embodiment, the receiving section 330 is configured to perform the analog-digital conversion upon the reception signals provided through the plurality of channels $CH_k$ from the ultrasound probe 310 to form the sampling data $S_{i,j}$, as shown in FIG. 8. The sampling data $S_{i,j}$ are stored in the storage unit 140. The receiving section 330 is further configured to set a sampling data set for selecting pixels that the sampling data $S_{i,j}$ are used as the pixel data thereof, during the reception beam-forming.

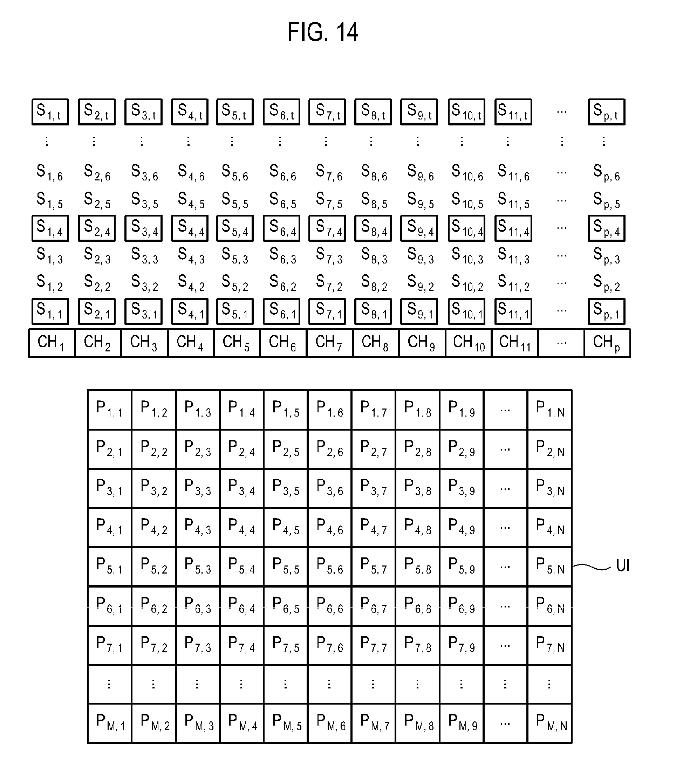

For example, the receiving section 330 is configured to set the sampling data $S_{1,1}$, $S_{1,4}$, ... $S_{1,t}$, $S_{2,1}$, $S_{2,4}$, ... $S_{2,t}$, ... $S_{p,t}$ as the sampling data set (denoted by a box) for selecting the pixels that the sampling data $S_{i,j}$ are used as the pixel data thereof, during the reception beam-forming, as shown in FIG. 14.

The receiving section 330 is further configured to detect the pixels corresponding to the respective sampling data of the sampling data set based on the positions of the elements 311 and the orientation of the respective pixels of the ultrasound image UI with respect to the elements 311. That is, the receiving section 330 selects the pixels that the respective sampling data of the sampling data set are used as the pixel data thereof, during the reception beam-forming based on the positions of the elements 311 and the orientation of the respective pixels of the ultrasound image UI with respect to the elements 311. The receiving section 330 is further configured to cumulatively assign the sampling data to the selected pixels in the same manner with the above embodiments. The receiving section 330 is further configured to perform the reception beam-forming upon the sampling data which are cumulatively assigned to the respective pixels of the ultrasound image UI to form the reception-focused data.

In yet another embodiment, the receiving section 330 is configured to perform a down-sampling upon the reception signals provided through the plurality of channels $CH_k$ from the ultrasound probe 310 to form down-sampling data. As described above, the receiving section 330 is further configured to detect the pixels corresponding to the respective sampling data, based on the positions of the elements 311 and the orientation of the respective pixels of the ultrasound image UI with respect to the elements 311. That is, the receiving section 330 selects the pixels that the respective sampling data are used as the pixel data thereof, during the reception beam-forming based on the positions of the elements 311 and the orientation of the pixels of the ultrasound image UI with respect to the elements 311. The receiving section 330 is further configured to cumulatively assign the respective sampling data to the selected pixels in the same manner of the above embodiments. The receiving section 330 is further configured to perform the reception beam-forming upon the sampling data which are cumulatively assigned to the respective pixels of the ultrasound image UI to form the reception-focused data.

However, it should be noted herein that the reception beam-forming may not be limited thereto.

Referring back to FIG. 3, the ultrasound data acquiring unit 120 further includes an ultrasound data forming section 340. The ultrasound data forming section 340 is configured to form the ultrasound data corresponding to the ultrasound image based on the reception-focused data provided from the receiving section 330. The ultrasound data forming section 340 is further configured to perform a signal process (e.g., gain control, etc) upon the reception-focused data.

In one embodiment, the ultrasound data forming section 340 is configured to form ultrasound data (hereinafter, referred to as "brightness mode ultrasound data") corresponding to the brightness mode image based on the brightness mode reception-focused data provided from the receiving section 330. The brightness mode ultrasound data include radio frequency data.

The ultrasound data forming section 340 is further configured to form ultrasound data (hereinafter, referred to as "Doppler mode ultrasound data") corresponding to the region of interest ROI based on the Doppler mode reception-focused data provided from the receiving section 330. The Doppler mode ultrasound data include in-phase/quadrature data. However, it should be noted herein that the Doppler mode ultrasound data may not be limited thereto.

For example, the ultrasound data forming section 340 forms first Doppler mode ultrasound data based on the first Doppler mode reception-focused data provided from the receiving section 330. The ultrasound data forming section 340 further forms second Doppler mode ultrasound data based on the second Doppler mode reception-focused data provided from the receiving section 330.

Referring back to FIG. 1, the ultrasound system 100 further includes a processing unit 130 in communication with the user input unit 110 and the ultrasound data acquiring unit 120. The processing unit 130 includes a central processing unit, a microprocessor, a graphic processing unit and the like.

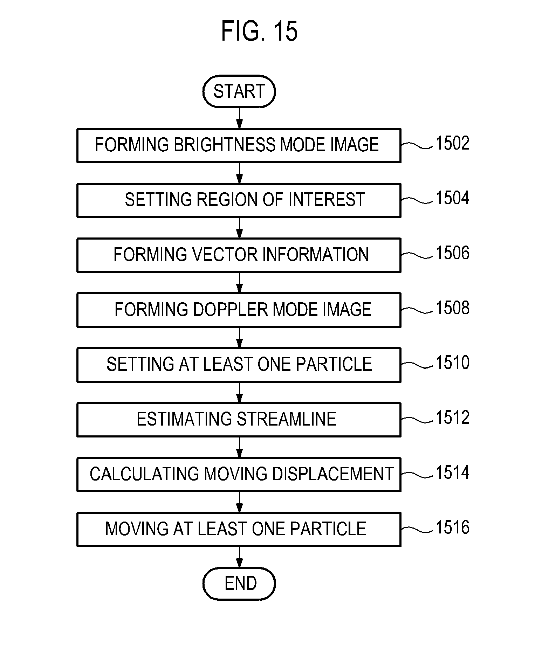

FIG. 15 is a flow chart showing a process of estimating motion of at least one particle. The processing unit 130 is configured to form the brightness mode image BI based on the brightness mode ultrasound data provided from the ultrasound data acquiring unit 120, at step S1502 in FIG. 15. The brightness mode image BI is displayed on a display unit 150. Thus, the user sets the region of interest ROI on the brightness mode image BI displayed on the display unit 150 by using the user input unit 110.

The processing unit 130 is configured to set the region of interest ROI on the brightness mode image BI based on the input information (i.e., first input information) provided from the user input unit 110, at step S1504 in FIG. 15. Thus, the ultrasound data acquiring unit 120 is configured to transmit the ultrasound signals to the living body and receive the ultrasound echo signals from the living body to acquire the Doppler mode ultrasound data, in consideration of the region of interest ROI.

The processing unit 130 is configured to form vector information based on the Doppler mode ultrasound data provided from the ultrasound data acquiring unit 120, at step S1506 in FIG. 15. That is, the processing unit 130 forms the vector information corresponding to motion (i.e., velocity and direction) of the target object based on the Doppler mode ultrasound data.

Generally, when the transmission direction of the ultrasound signals is equal to the reception direction of the ultrasound echo signals and a Doppler angle is $\theta$, the following relationship is established:

$$X \cos \theta = \frac{C_0 f_d}{2 f_0} \qquad (1)$$

In Equation 1, X represents a reflector velocity (i.e., velocity of target object), $C_0$ represents a sound speed in the living body, $f_d$ represents a Doppler shift frequency, and $f_0$ represents an ultrasound frequency.

The Doppler shift frequency $f_d$ is calculated by the difference between a frequency of the ultrasound signals (i.e., transmission beam) and a frequency of the ultrasound echo signals (i.e., reception beam). Also, the velocity component $X\cos\theta$ projected to the transmission direction is calculated by Equation 1.

When the transmission direction of the ultrasound signals (i.e., transmission beam) is different from the reception direction of the ultrasound echo signals (i.e., reception beam), the following relationship is established:

$$X \cos \theta_T + X \cos \theta_R = \frac{C_0 f_d}{f_0} \qquad (2)$$

In Equation 2, $\theta_T$ represents an angle between the ultrasound signals (i.e., transmission beam) and the blood flow, and $\theta_R$ represents an angle between the ultrasound echo signals (i.e., reception beam) and the blood flow.

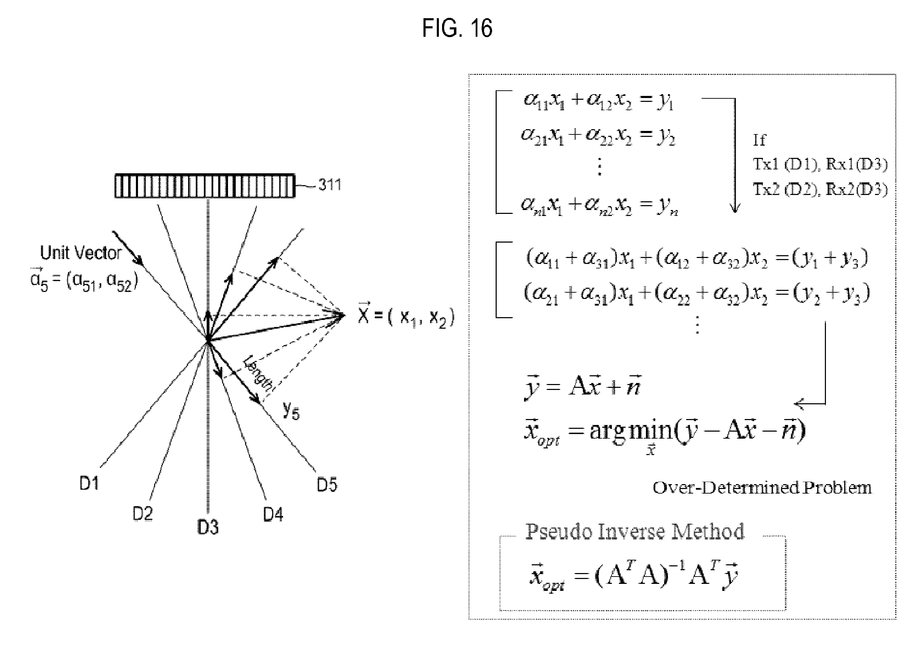

FIG. 16 is a schematic diagram showing an example of the transmission directions, the reception directions, the vector information and an over-determined problem. Referring to FIG. 16, when the ultrasound signals (i.e., transmission beam) are transmitted in a first direction D1 and the ultrasound echo signals (i.e., reception beam) are received in the first direction D1, the following relationship is established:

$$\vec{\alpha_1} \vec{X} = \alpha_{11} x_1 + \alpha_{12} x_2 = y_1 = X \cos \theta \qquad (3)$$

In Equation 3, $\vec{\alpha_1} = (\alpha_{11}, \alpha_{12})$ represents a unit vector of the first direction D1, $\vec{X} = (x_1, x_2)$ represents variables, and $y_1$ is calculated by Equation 1.

When the ultrasound signals (i.e., transmission beam) are transmitted in a second direction D2 and the ultrasound echo signals (i.e., reception beam) are received in a third direction D3, the following relationship is established:

$$(\alpha_{21} + \alpha_{31}) x_1 + (\alpha_{22} + \alpha_{32}) x_2 = (y_2 + y_3) = X \cos \theta_2 + X \cos \theta_3 \qquad (4)$$

Equations 3 and 4 are set to assume two-dimensional environment. Further, Equations 3 and 4 are expanded to three-dimensional environment. That is, when expanding Equations 3 and 4 to the three-dimensional environment, the following relationship is established:

$$\alpha_{11}x_1 + \alpha_{12}x_2 + \alpha_{13}x_3 = y \qquad (5)$$

In the case of the two-dimensional environment (i.e., two-dimensional vector), at least two equations are required to calculate the variables $x_1$ and $x_2$. For example, when the ultrasound signals (i.e., transmission beam) are transmitted in the third direction D3 and the ultrasound echo signals (i.e., reception beam) are received in the second direction D2 and a fourth direction D4 as shown in FIG. 16, the following equations is established:

$$(\alpha_{31} + \alpha_{21})x_1 + (\alpha_{32} + \alpha_{22})x_2 = (y_3 + y_2)$$

$$(\alpha_{31} + \alpha_{41})x_1 + (\alpha_{32} + \alpha_{42})x_2 = (y_3 + y_4) \qquad (6)$$

The vector $\vec{X} = (x_1, x_2)$ is calculated by the equations of Equation 6.

When the reception beam-forming is performed in at least two angles (i.e., at least two reception directions), at least two equations are obtained and represented as the over-determined problem, as shown in FIG. 16. The over-determined problem is solved by a pseudo inverse method, a weighted least square method and the like based on noise characteristics added to the Doppler shift frequency. The over-determined problem is well known in the art. Thus, it has not been described in detail so as not to unnecessarily obscure the present disclosure. That is, $M \times N$ equations are obtained by M transmission directions and the reception beam-forming of N reception directions at every transmission.

Referring back to FIG. 15, the processing unit 130 is configured to form the Doppler mode image $DMI_k$, wherein the k is a positive integer, based on the vector information, at step S1508 in FIG. 15. The Doppler mode image $DMI_k$ is displayed on the display unit 150. Thus, the user sets the at least one particle on the Doppler mode image $DMI_k$ displayed on the display unit 150 by using the user input unit 110.

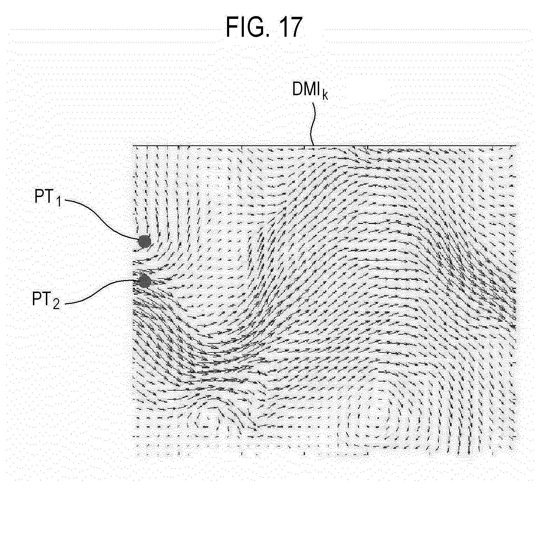

The processing unit 130 is configured to set the at least one particle on the Doppler mode image $DMI_k$ based on the input information (i.e., second input information) provided from the user input unit 110, at step S1510 in FIG. 15. That is, the processing unit 130 sets the at least one particle on the Doppler mode image $DMI_k$ based on the second input information for setting the number, position, size, color and shape of the particle, an alignment position between the particles and the like. For example, the processing unit 130 sets the particles $PT_1$, $PT_2$ on the Doppler mode image $DMI_k$ based on the second input information provided from the user input unit 110, as shown in FIG. 17.

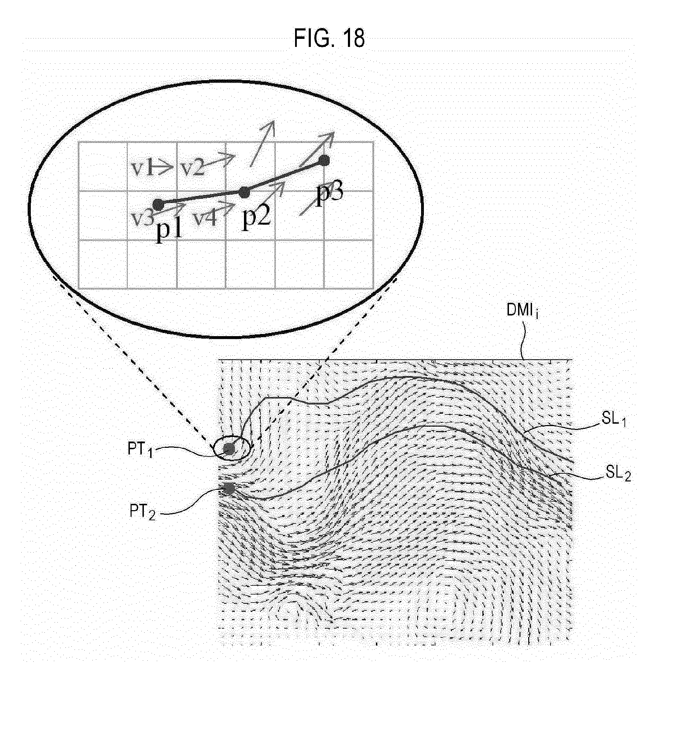

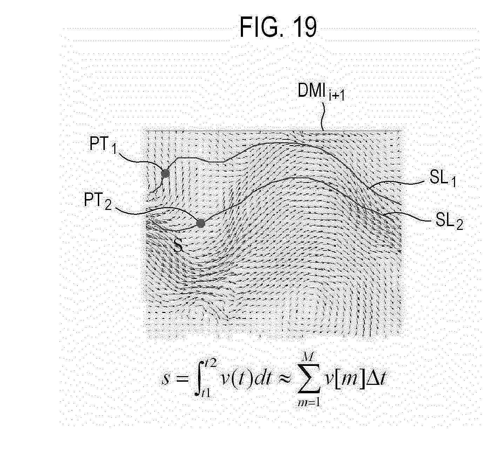

The processing unit 130 is configured to estimate at least one streamline corresponding to motion of the at least one particle based on the vector information, at step S1512 in FIG. 15. For example, the processing unit 130 interpolates vector information v1, v2, v3 and v4 adjacent to a first position p1 of the first particle $PT_1$ for an $i^{th}$ Doppler mode image $DMI_i$, wherein the i is a positive integer, to estimate a stream direction at the first position p1, as shown in FIG. 18. The processing unit 130 further estimates a second position p2 of the first particle $PT_1$ based on the stream direction and a predetermined moving displacement S with respect to the first position p1. The processing unit 130 further interpolates vector information adjacent to the second position p2 of the first particle $PT_1$ to estimate a stream direction at the second position p2. The processing unit 130 further estimates a third position p3 of the first particle PT1 based on the stream direction at the second position p2 and the predetermined moving displacement S with respect to the second position p2. As described above, the processing unit 130 estimates a first streamline $SL_1$ corresponding to the first particle $PT_1$ and a second streamline $SL_2$ corresponding to the second particle $PT_2$ for the $i^{th}$ Doppler mode image $DMI_i$. Thereafter, the processing unit 130 estimates the first streamline $SL_1$ corresponding to the first particle $PT_1$ and the second streamline $SL_2$ corresponding to the second particle $PT_2$ for an $(i+1)^{th}$ Doppler mode image $DMI_{i+1}$, as shown in FIG. 19.

Referring back to FIG. 15, the processing unit 130 is configured to calculate a moving displacement of the at least one particle based on a time interval between adjacent Doppler mode images and the vector information (i.e., current velocity of at least one particle), at step S1514 in FIG. 15. For example, the processing unit 130 calculates the moving displacement ($s=v \times dt$) of the respective particles $PT_1$, $PT_2$ based on the time interval $dt$ between the Doppler mode images $DMI_i$ and $DMI_{i+1}$ and the velocity $v$ at the current position of the respective particles $PT_1$, $PT_2$.

Optionally, the processing unit 130 further calculates the moving displacement of the at least one particle in inverse proportion to a weight corresponding to the at least one particle, wherein the weight is set by a user.

The processing unit 130 is configured to move the at least one particle by a distance corresponding to the calculated moving displacement along the streamline set on the Doppler mode image $DMI_k$, at step S1516 in FIG. 15. For example, the processing unit 130 moves the particle $PT_1$ along the first streamline $SL_1$ by the distance corresponding to the moving displacement of the particle $PT_1$ for the $i^{th}$ Doppler mode image $DMI_{i+1}$, as shown in FIG. 19. The processing unit 130 further moves the particle $PT_2$ along the second streamline $SL_2$ by the distance corresponding to the moving displacement of the particle $PT_2$ for the Doppler mode image $DMI_{i+1}$, as shown in FIG. 19.

In another embodiment, the processing unit 130 readjusts the number and position of the particle at every Doppler mode image $DMI_k$. For example, the processing unit 130 sets the N particles on the $i^{th}$ Doppler mode image $DMI_i$, wherein the N is an integer two or greater. The processing unit 130 further detects at least one particle, which does not move (i.e., moving displacement is 0), from the $(i+1)^{th}$ Doppler mode image $DMI_{i+1}$. The processing unit 130 further removes the detected particle. The processing unit further sets the number of a new particle corresponding to the removed particle on the $(i+1)^{th}$ Doppler mode image $DMI_{i+1}$. Thus, the N particles are set on the Doppler mode image $DMI_k$.

In yet another embodiment, the processing unit 130 relocates the position of the at least one particle on a line at every predetermined frame (i.e., Doppler mode image). In this way, a profile pattern corresponding to blood flow is provided by relocating the position of the at least one particle on the line.

In still yet another embodiment, the processing unit 130 sets the at least one particle on the Doppler mode image $DMI_i$ in synchronization with electrocardiogram signals. In this way, the motion of the at least one particle is provided in synchronization with the electrocardiogram signals by setting the at least one particle in synchronization with the electrocardiogram signals.

In a further embodiment, the processing unit 130 estimates the streamline corresponding to the at least one particle between the $i^{th}$ Doppler mode image $DMI_i$ and the $(i+1)_{th}$ Doppler mode image $DMI_{i+1}$. That is, the processing unit 130 further estimates the streamline by interpolating a vector field of the $i^{th}$ Doppler mode image $DMI_i$ and a vector field of the $(i+1)^{th}$ Doppler mode image $DMI_{i+1}$.

In a still yet further embodiment, the processing unit 130 estimates the motion of the at least one particle at every frame (i.e., Doppler mode image) or between the $i^{th}$ Doppler mode image $DMI_i$ and the $(i+1)^{th}$ Doppler mode image $DMI_{i+1}$.

Referring back to FIG. 1, the ultrasound system 100 further includes the storage unit 140. The storage unit 140 stores the ultrasound data (i.e., brightness mode ultrasound data and Doppler mode ultrasound data) acquired by the ultrasound data acquiring unit 120. The storage unit 140 further stores the vector information formed by the processing unit 130.

The ultrasound system 100 further includes the display unit 150. The display unit 150 is configured to display the brightness mode image BI formed by the processing unit 130. The display unit 150 is further configured to display the Doppler mode image formed by the processing unit 130. The display unit 150 is further configured to display the at least one particle.

Although embodiments have been described with reference to a number of illustrative embodiments thereof, it should be understood that numerous other modifications and embodiments can be devised by those skilled in the art that will fall within the scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

**Claims**

1. An ultrasound system, comprising:

   a processing unit configured to form vector information of a target object based on ultrasound data corresponding to the target object, form a plurality of Doppler mode images based on the vector information, set at least one particle on the Doppler mode images based on input information of a user, and estimate a motion of the at least one particle based on the vector information.

2. The ultrasound system of Claim 1, wherein the processing unit is configured to:

   form the vector information corresponding to a velocity and a direction of the target object in consideration of at least one transmission direction and at least one reception direction corresponding to the at least one transmission direction based on the ultrasound data;
   interpolate the vector information adjacent to an $i^{th}$ position of the at least one particle to estimate a stream direction at the $i^{th}$ position, wherein the i is a positive integer;

estimate an $(i+1)^{th}$ position of the at least one particle based on the stream direction and a predetermined moving displacement with respect to the $i^{th}$ position to estimate a streamline corresponding to a motion of the at least one particle based on the vector information;

calculate a moving displacement based on the time interval between an $i^{th}$ Doppler mode image and an $(i+1)^{th}$ Doppler mode image and a velocity at a current position of the at least one particle; and

move the at least one particle along the streamline by a distance corresponding to the moving displacement.

3. The ultrasound system of Claim 2, wherein the processing unit is further configured to calculate the moving displacement in an inverse proportion to a weight corresponding to the at least one particle.

4. The ultrasound system of Claim 3, wherein the processing unit is further configured to readjust the number and position of the particle at every Doppler mode image.

5. The ultrasound system of Claim 4, wherein the processing unit is further configured to:

detect at least one non-moving particle from the $i^{th}$ Doppler mode image;

remove the detected particle from the $(i+1)^{th}$ Doppler mode image;

set the number of new particle corresponding to the removed particle on the $(i+1)^{th}$ Doppler mode image.

6. The ultrasound system of Claim 1, wherein the processing unit is further configured to relocate the position of the at least one particle on a line at every predetermined Doppler mode image, and

wherein the processing unit is further configured to set the at least one particle on the Doppler mode images in synchronization with electrocardiogram signals.

7. The ultrasound system of Claim 2, wherein the processing unit is further configured to estimate the streamline corresponding to the at least one particle between the $i^{th}$ Doppler mode image and the $(i+1)^{th}$ Doppler mode image.

8. The ultrasound system of Claim 2, wherein the processing unit is further configured to estimate motion of the at least one particle at every Doppler mode image or between an $i^{th}$ Doppler mode image and an $(i+1)^{th}$ Doppler mode image.

9. A method of estimating motion of at least one particle, comprising:

a) forming vector information of a target object based on ultrasound data corresponding to the target object;

b) forming a plurality of Doppler mode images based on the vector information;

c) setting at least one particle on the Doppler mode images based on input information of a user; and

d) estimating a motion of the at least one particle based on the vector information.

10. The method of Claim 10, wherein the step a) comprises:

forming the vector information corresponding to a velocity and a direction of the target object in consideration of at least one transmission direction and at least one reception direction corresponding to the at least one transmission direction based on the ultrasound data, and

wherein the step d) comprises:

interpolating the vector information adjacent to an $i^{th}$ position of the at least one particle to estimate a stream direction at the $i^{th}$ position, wherein the i is a positive integer;

estimating an $(i+1)^{th}$ position of the at least one particle based on the stream direction and a predetermined moving displacement with respect to the $i^{th}$ position to estimate a streamline corresponding to a motion of the at least one particle based on the vector information;

calculating the moving displacement based on the time interval between an $i^{th}$ Doppler mode image and an $(i+1)^{th}$ Doppler mode image and a velocity at a current position of the at least one particle; and

moving the at least one particle along the streamline by a distance corresponding to the moving displacement.

11. The method of Claim 10, wherein the step d) further comprises:

calculating the moving displacement in an inverse proportion to a weight corresponding to the at least one particle, wherein the weight is set by a user.

12. The method of Claim 10, further comprising:

e) readjusting the number and position of the particle at every Doppler mode image, and
wherein the step e) further comprises:
detecting at least one non-moving particle from the i$^{th}$ Doppler mode image;
removing the detected particle from the (i+1)$^{th}$ Doppler mode image;
setting the number of new particle corresponding to the removed particle on the (i+1)$^{th}$ Doppler mode image.

13. The method of Claim 10, further comprising:

e) relocating the position of the at least one particle on a line at every predetermined Doppler mode image.

14. The method of Claim 10, wherein the step c) further comprises:

setting the at least one particle on the Doppler mode images in synchronization with electrocardiogram signals and

wherein the step d) further comprises:

estimating the streamline corresponding to the at least one particle between the i$^{th}$ Doppler mode image and the (i+1)$^{th}$ Doppler mode image.

15. The method of Claim 10, wherein the step d) further comprises:

estimating motion of the at least one particle at every Doppler mode image or between an i$^{th}$ Doppler mode image and an (i+1)$^{th}$ Doppler mode image.

FIG. 1

100

110

USER
INPUT UNIT

120

ULTRASOUND
DATA ACQUIRING
UNIT

130

PROCESSING
UNIT

150

DISPLAY
UNIT

140

STORAGE
UNIT

## FIG. 2

## FIG. 3

FIG. 4

311

Rx$_2$     Rx$_1$

Tx

## FIG. 5

311

Rx$_1$, Rx$_2$

Tx$_1$

Tx$_2$

Tx$_1$ (ensemble number)

Tx$_2$ (ensemble number)

PRI

...

...

time

# FIG. 6

# FIG. 7

# FIG. 8

| $S_{1,t}$ | $S_{2,t}$ | $S_{3,t}$ | $S_{4,t}$ | $S_{5,t}$ | $S_{6,t}$ | $S_{7,t}$ | $S_{8,t}$ | $S_{9,t}$ | $S_{10,t}$ | $S_{11,t}$ | $\cdots$ | $S_{p,t}$ |
|---|---|---|---|---|---|---|---|---|---|---|---|---|
| $\vdots$ | $\vdots$ | $\vdots$ | $\vdots$ | $\vdots$ | $\vdots$ | $\vdots$ | $\vdots$ | $\vdots$ | $\vdots$ | $\vdots$ | $\vdots$ | $\vdots$ |
| $S_{1,6}$ | $S_{2,6}$ | $S_{3,6}$ | $S_{4,6}$ | $S_{5,6}$ | $S_{6,6}$ | $S_{7,6}$ | $S_{8,6}$ | $S_{9,6}$ | $S_{10,6}$ | $S_{11,6}$ | $\cdots$ | $S_{p,6}$ |
| $S_{1,5}$ | $S_{2,5}$ | $S_{3,5}$ | $S_{4,5}$ | $S_{5,5}$ | $S_{6,5}$ | $S_{7,5}$ | $S_{8,5}$ | $S_{9,5}$ | $S_{10,5}$ | $S_{11,5}$ | $\cdots$ | $S_{p,5}$ |
| $S_{1,4}$ | $S_{2,4}$ | $S_{3,4}$ | $S_{4,4}$ | $S_{5,4}$ | $S_{6,4}$ | $S_{7,4}$ | $S_{8,4}$ | $S_{9,4}$ | $S_{10,4}$ | $S_{11,4}$ | $\cdots$ | $S_{p,4}$ |
| $S_{1,3}$ | $S_{2,3}$ | $S_{3,3}$ | $S_{4,3}$ | $S_{5,3}$ | $S_{6,3}$ | $S_{7,3}$ | $S_{8,3}$ | $S_{9,3}$ | $S_{10,3}$ | $S_{11,3}$ | $\cdots$ | $S_{p,3}$ |
| $S_{1,2}$ | $S_{2,2}$ | $S_{3,2}$ | $S_{4,2}$ | $S_{5,2}$ | $S_{6,2}$ | $S_{7,2}$ | $S_{8,2}$ | $S_{9,2}$ | $S_{10,2}$ | $S_{11,2}$ | $\cdots$ | $S_{p,2}$ |
| $S_{1,1}$ | $S_{2,1}$ | $S_{3,1}$ | $S_{4,1}$ | $S_{5,1}$ | $S_{6,1}$ | $S_{7,1}$ | $S_{8,1}$ | $S_{9,1}$ | $S_{10,1}$ | $S_{11,1}$ | $\cdots$ | $S_{p,1}$ |
| $CH_1$ | $CH_2$ | $CH_3$ | $CH_4$ | $CH_5$ | $CH_6$ | $CH_7$ | $CH_8$ | $CH_9$ | $CH_{10}$ | $CH_{11}$ | $\cdots$ | $CH_p$ |

| $P_{1,1}$ | $P_{1,2}$ | $P_{1,3}$ | $P_{1,4}$ | $P_{1,5}$ | $P_{1,6}$ | $P_{1,7}$ | $P_{1,8}$ | $P_{1,9}$ | $\cdots$ | $P_{1,N}$ |
|---|---|---|---|---|---|---|---|---|---|---|
| $P_{2,1}$ | $P_{2,2}$ | $P_{2,3}$ | $P_{2,4}$ | $P_{2,5}$ | $P_{2,6}$ | $P_{2,7}$ | $P_{2,8}$ | $P_{2,9}$ | $\cdots$ | $P_{2,N}$ |
| $P_{3,1}$ | $P_{3,2}$ | $P_{3,3}$ | $P_{3,4}$ | $P_{3,5}$ | $P_{3,6}$ | $P_{3,7}$ | $P_{3,8}$ | $P_{3,9}$ | $\cdots$ | $P_{3,N}$ |
| $P_{4,1}$ | $P_{4,2}$ | $P_{4,3}$ | $P_{4,4}$ | $P_{4,5}$ | $P_{4,6}$ | $P_{4,7}$ | $P_{4,8}$ | $P_{4,9}$ | $\cdots$ | $P_{4,N}$ |
| $P_{5,1}$ | $P_{5,2}$ | $P_{5,3}$ | $P_{5,4}$ | $P_{5,5}$ | $P_{5,6}$ | $P_{5,7}$ | $P_{5,8}$ | $P_{5,9}$ | $\cdots$ | $P_{5,N}$ |
| $P_{6,1}$ | $P_{6,2}$ | $P_{6,3}$ | $P_{6,4}$ | $P_{6,5}$ | $P_{6,6}$ | $P_{6,7}$ | $P_{6,8}$ | $P_{6,9}$ | $\cdots$ | $P_{6,N}$ |
| $P_{7,1}$ | $P_{7,2}$ | $P_{7,3}$ | $P_{7,4}$ | $P_{7,5}$ | $P_{7,6}$ | $P_{7,7}$ | $P_{7,8}$ | $P_{7,9}$ | $\cdots$ | $P_{7,N}$ |
| $\vdots$ | $\vdots$ | $\vdots$ | $\vdots$ | $\vdots$ | $\vdots$ | $\vdots$ | $\vdots$ | $\vdots$ | $\vdots$ | $\vdots$ |
| $P_{M,1}$ | $P_{M,2}$ | $P_{M,3}$ | $P_{M,4}$ | $P_{M,5}$ | $P_{M,6}$ | $P_{M,7}$ | $P_{M,8}$ | $P_{M,9}$ | $\cdots$ | $P_{M,N}$ |

UI

# FIG. 9

# FIG. 10

| | | | | | | | | | | |
|---|---|---|---|---|---|---|---|---|---|---|
| | | | | | | | | | | |
| | | | | | | | | | | |
| $S_{6,3}$ | $S_{6,3}$ | | | | | | | | | $S_{6,3}$ |
| | $S_{6,3}$ | $S_{6,3}$ | $S_{6,3}$ | $S_{6,3}$ | $S_{6,3}$ | $S_{6,3}$ | $S_{6,3}$ | $S_{6,3}$ | ... | |
| | | | | | | | | | | |
| | | | | | | | | | | |
| | | | | | | | | | | |
| | | | | | | | | | | |
| | | | | | | | | | | |

UI

# FIG. 11

| $S_{1,t}$ | $S_{2,t}$ | $S_{3,t}$ | $S_{4,t}$ | $S_{5,t}$ | $S_{6,t}$ | $S_{7,t}$ | $S_{8,t}$ | $S_{9,t}$ | $S_{10,t}$ | $S_{11,t}$ | $\cdots$ | $S_{p,t}$ |
|---|---|---|---|---|---|---|---|---|---|---|---|---|
| $\vdots$ | $\vdots$ | $\vdots$ | $\vdots$ | $\vdots$ | $\vdots$ | $\vdots$ | $\vdots$ | $\vdots$ | $\vdots$ | $\vdots$ | $\vdots$ | $\vdots$ |
| $S_{1,6}$ | $S_{2,6}$ | $S_{3,6}$ | $S_{4,6}$ | $S_{5,6}$ | $S_{6,6}$ | $S_{7,6}$ | $S_{8,6}$ | $S_{9,6}$ | $S_{10,6}$ | $S_{11,6}$ | $\cdots$ | $S_{p,6}$ |
| $S_{1,5}$ | $S_{2,5}$ | $S_{3,5}$ | $S_{4,5}$ | $S_{5,5}$ | $S_{6,5}$ | $S_{7,5}$ | $S_{8,5}$ | $S_{9,5}$ | $S_{10,5}$ | $S_{11,5}$ | $\cdots$ | $S_{p,5}$ |
| $S_{1,4}$ | $S_{2,4}$ | $S_{3,4}$ | $S_{4,4}$ | $S_{5,4}$ | $S_{6,4}$ | $S_{7,4}$ | $S_{8,4}$ | $S_{9,4}$ | $S_{10,4}$ | $S_{11,4}$ | $\cdots$ | $S_{p,4}$ |
| $S_{1,3}$ | $S_{2,3}$ | $S_{3,3}$ | $S_{4,3}$ | $S_{5,3}$ | $S_{6,3}$ | $S_{7,3}$ | $S_{8,3}$ | $S_{9,3}$ | $S_{10,3}$ | $S_{11,3}$ | $\cdots$ | $S_{p,3}$ |
| $S_{1,2}$ | $S_{2,2}$ | $S_{3,2}$ | $S_{4,2}$ | $S_{5,2}$ | $S_{6,2}$ | $S_{7,2}$ | $S_{8,2}$ | $S_{9,2}$ | $S_{10,2}$ | $S_{11,2}$ | $\cdots$ | $S_{p,2}$ |
| $S_{1,1}$ | $S_{2,1}$ | $S_{3,1}$ | $S_{4,1}$ | $S_{5,1}$ | $S_{6,1}$ | $S_{7,1}$ | $S_{8,1}$ | $S_{9,1}$ | $S_{10,1}$ | $S_{11,1}$ | $\cdots$ | $S_{p,1}$ |
| $CH_1$ | $CH_2$ | $CH_3$ | $CH_4$ | $CH_5$ | $CH_6$ | $CH_7$ | $CH_8$ | $CH_9$ | $CH_{10}$ | $CH_{11}$ | $\cdots$ | $CH_p$ |

| $P_{1,1}$ | $P_{1,2}$ | $P_{1,3}$ | $P_{1,4}$ | $P_{1,5}$ | $P_{1,6}$ | $P_{1,7}$ | $P_{1,8}$ | $P_{1,9}$ | $\cdots$ | $P_{1,N}$ |
|---|---|---|---|---|---|---|---|---|---|---|
| $P_{2,1}$ | $P_{2,2}$ | $P_{2,3}$ | $P_{2,4}$ | $P_{2,5}$ | $P_{2,6}$ | $P_{2,7}$ | $P_{2,8}$ | $P_{2,9}$ | $\cdots$ | $P_{2,N}$ |
| $P_{3,1}$ | $P_{3,2}$ | $P_{3,3}$ | $P_{3,4}$ | $P_{3,5}$ | $P_{3,6}$ | $P_{3,7}$ | $P_{3,8}$ | $P_{3,9}$ | $\cdots$ | $P_{3,N}$ |
| $P_{4,1}$ | $P_{4,2}$ | $P_{4,3}$ | $P_{4,4}$ | $P_{4,5}$ | $P_{4,6}$ | $P_{4,7}$ | $P_{4,8}$ | $P_{4,9}$ | $\cdots$ | $P_{4,N}$ |
| $P_{5,1}$ | $P_{5,2}$ | $P_{5,3}$ | $P_{5,4}$ | $P_{5,5}$ | $P_{5,6}$ | $P_{5,7}$ | $P_{5,8}$ | $P_{5,9}$ | $\cdots$ | $P_{5,N}$ |
| $P_{6,1}$ | $P_{6,2}$ | $P_{6,3}$ | $P_{6,4}$ | $P_{6,5}$ | $P_{6,6}$ | $P_{6,7}$ | $P_{6,8}$ | $P_{6,9}$ | $\cdots$ | $P_{6,N}$ |
| $P_{7,1}$ | $P_{7,2}$ | $P_{7,3}$ | $P_{7,4}$ | $P_{7,5}$ | $P_{7,6}$ | $P_{7,7}$ | $P_{7,8}$ | $P_{7,9}$ | $\cdots$ | $P_{7,N}$ |
| $\vdots$ | $\vdots$ | $\vdots$ | $\vdots$ | $\vdots$ | $\vdots$ | $\vdots$ | $\vdots$ | $\vdots$ | $\vdots$ | $\vdots$ |
| $P_{M,1}$ | $P_{M,2}$ | $P_{M,3}$ | $P_{M,4}$ | $P_{M,5}$ | $P_{M,6}$ | $P_{M,7}$ | $P_{M,8}$ | $P_{M,9}$ | $\cdots$ | $P_{M,N}$ |

$CV_{6,4}$

UI

FIG. 12

# FIG. 13

$P_{3,2}$

$W_1$

$CV_{6,3}$

$W_2$

$P_{4,2}$

$\alpha_1 \, S_{6,3}$

$\alpha_2 \, S_{6,3}$

# FIG. 14

| $S_{1,t}$ | $S_{2,t}$ | $S_{3,t}$ | $S_{4,t}$ | $S_{5,t}$ | $S_{6,t}$ | $S_{7,t}$ | $S_{8,t}$ | $S_{9,t}$ | $S_{10,t}$ | $S_{11,t}$ | $\cdots$ | $S_{p,t}$ |
|---|---|---|---|---|---|---|---|---|---|---|---|---|

$S_{1,6}$ $S_{2,6}$ $S_{3,6}$ $S_{4,6}$ $S_{5,6}$ $S_{6,6}$ $S_{7,6}$ $S_{8,6}$ $S_{9,6}$ $S_{10,6}$ $S_{11,6}$ $\cdots$ $S_{p,6}$

$S_{1,5}$ $S_{2,5}$ $S_{3,5}$ $S_{4,5}$ $S_{5,5}$ $S_{6,5}$ $S_{7,5}$ $S_{8,5}$ $S_{9,5}$ $S_{10,5}$ $S_{11,5}$ $\cdots$ $S_{p,5}$

| $S_{1,4}$ | $S_{2,4}$ | $S_{3,4}$ | $S_{4,4}$ | $S_{5,4}$ | $S_{6,4}$ | $S_{7,4}$ | $S_{8,4}$ | $S_{9,4}$ | $S_{10,4}$ | $S_{11,4}$ | $\cdots$ | $S_{p,4}$ |
|---|---|---|---|---|---|---|---|---|---|---|---|---|

$S_{1,3}$ $S_{2,3}$ $S_{3,3}$ $S_{4,3}$ $S_{5,3}$ $S_{6,3}$ $S_{7,3}$ $S_{8,3}$ $S_{9,3}$ $S_{10,3}$ $S_{11,3}$ $\cdots$ $S_{p,3}$

$S_{1,2}$ $S_{2,2}$ $S_{3,2}$ $S_{4,2}$ $S_{5,2}$ $S_{6,2}$ $S_{7,2}$ $S_{8,2}$ $S_{9,2}$ $S_{10,2}$ $S_{11,2}$ $\cdots$ $S_{p,2}$

| $S_{1,1}$ | $S_{2,1}$ | $S_{3,1}$ | $S_{4,1}$ | $S_{5,1}$ | $S_{6,1}$ | $S_{7,1}$ | $S_{8,1}$ | $S_{9,1}$ | $S_{10,1}$ | $S_{11,1}$ | $\cdots$ | $S_{p,1}$ |
|---|---|---|---|---|---|---|---|---|---|---|---|---|
| $CH_1$ | $CH_2$ | $CH_3$ | $CH_4$ | $CH_5$ | $CH_6$ | $CH_7$ | $CH_8$ | $CH_9$ | $CH_{10}$ | $CH_{11}$ | $\cdots$ | $CH_p$ |

| $P_{1,1}$ | $P_{1,2}$ | $P_{1,3}$ | $P_{1,4}$ | $P_{1,5}$ | $P_{1,6}$ | $P_{1,7}$ | $P_{1,8}$ | $P_{1,9}$ | $\cdots$ | $P_{1,N}$ |
|---|---|---|---|---|---|---|---|---|---|---|
| $P_{2,1}$ | $P_{2,2}$ | $P_{2,3}$ | $P_{2,4}$ | $P_{2,5}$ | $P_{2,6}$ | $P_{2,7}$ | $P_{2,8}$ | $P_{2,9}$ | $\cdots$ | $P_{2,N}$ |
| $P_{3,1}$ | $P_{3,2}$ | $P_{3,3}$ | $P_{3,4}$ | $P_{3,5}$ | $P_{3,6}$ | $P_{3,7}$ | $P_{3,8}$ | $P_{3,9}$ | $\cdots$ | $P_{3,N}$ |
| $P_{4,1}$ | $P_{4,2}$ | $P_{4,3}$ | $P_{4,4}$ | $P_{4,5}$ | $P_{4,6}$ | $P_{4,7}$ | $P_{4,8}$ | $P_{4,9}$ | $\cdots$ | $P_{4,N}$ |
| $P_{5,1}$ | $P_{5,2}$ | $P_{5,3}$ | $P_{5,4}$ | $P_{5,5}$ | $P_{5,6}$ | $P_{5,7}$ | $P_{5,8}$ | $P_{5,9}$ | $\cdots$ | $P_{5,N}$ |
| $P_{6,1}$ | $P_{6,2}$ | $P_{6,3}$ | $P_{6,4}$ | $P_{6,5}$ | $P_{6,6}$ | $P_{6,7}$ | $P_{6,8}$ | $P_{6,9}$ | $\cdots$ | $P_{6,N}$ |
| $P_{7,1}$ | $P_{7,2}$ | $P_{7,3}$ | $P_{7,4}$ | $P_{7,5}$ | $P_{7,6}$ | $P_{7,7}$ | $P_{7,8}$ | $P_{7,9}$ | $\cdots$ | $P_{7,N}$ |
| $\vdots$ | $\vdots$ | $\vdots$ | $\vdots$ | $\vdots$ | $\vdots$ | $\vdots$ | $\vdots$ | $\vdots$ | $\vdots$ | $\vdots$ |
| $P_{M,1}$ | $P_{M,2}$ | $P_{M,3}$ | $P_{M,4}$ | $P_{M,5}$ | $P_{M,6}$ | $P_{M,7}$ | $P_{M,8}$ | $P_{M,9}$ | $\cdots$ | $P_{M,N}$ |

UI

# FIG. 15

```
                    ┌─────────┐
                    │  START  │
                    └─────────┘
                         │
                         ▼
    ┌────────────────────────────────────────┐
    │      FORMING BRIGHTNESS MODE IMAGE       │───1502
    └────────────────────────────────────────┘
                         │
                         ▼
    ┌────────────────────────────────────────┐
    │        SETTING REGION OF INTEREST        │───1504
    └────────────────────────────────────────┘
                         │
                         ▼
    ┌────────────────────────────────────────┐
    │        FORMING VECTOR INFORMATION        │───1506
    └────────────────────────────────────────┘
                         │
                         ▼
    ┌────────────────────────────────────────┐
    │        FORMING DOPPLER MODE IMAGE        │───1508
    └────────────────────────────────────────┘
                         │
                         ▼
    ┌────────────────────────────────────────┐
    │       SETTING AT LEAST ONE PARTICLE      │───1510
    └────────────────────────────────────────┘
                         │
                         ▼
    ┌────────────────────────────────────────┐
    │           ESTIMATING STREAMLINE          │───1512
    └────────────────────────────────────────┘
                         │
                         ▼
    ┌────────────────────────────────────────┐
    │     CALCULATING MOVING DISPLACEMENT      │───1514
    └────────────────────────────────────────┘
                         │
                         ▼
    ┌────────────────────────────────────────┐
    │       MOVING AT LEAST ONE PARTICLE       │───1516
    └────────────────────────────────────────┘
                         │
                         ▼
                    ┌─────────┐
                    │   END   │
                    └─────────┘
```

## FIG. 16

Unit Vector
$\vec{a}_5 = (a_{51}, a_{52})$

$\vec{X} = (x_1, x_2)$

Length

$y_5$

D1  D2  D3  D4  D5

311

$$\alpha_{11}x_1 + \alpha_{12}x_2 = y_1$$
$$\alpha_{21}x_1 + \alpha_{22}x_2 = y_2$$
$$\vdots$$
$$\alpha_{n1}x_1 + \alpha_{n2}x_2 = y_n$$

If
Tx1 (D1), Rx1(D3)
Tx2 (D2), Rx2(D3)

$$(\alpha_{11} + \alpha_{31})x_1 + (\alpha_{12} + \alpha_{32})x_2 = (y_1 + y_3)$$
$$(\alpha_{21} + \alpha_{31})x_1 + (\alpha_{22} + \alpha_{32})x_2 = (y_2 + y_3)$$
$$\vdots$$

$$\vec{y} = A\vec{x} + \vec{n}$$

$$\vec{x}_{opt} = \arg\min_{\vec{x}}(\vec{y} - A\vec{x} - \vec{n})$$

Over-Determined Problem

Pseudo Inverse Method

$$\vec{x}_{opt} = (A^T A)^{-1} A^T \vec{y}$$

FIG. 17

FIG. 18

## FIG. 19

$$s = \int_{t1}^{t2} v(t)dt \approx \sum_{m=1}^{M} v[m]\Delta t$$

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020110144489